(19) Europäisches Patentamt

European Patent Office

Office européen des brevets

(11) **EP 2 742 853 B1**

(12) **EUROPEAN PATENT SPECIFICATION**

(45) Date of publication and mention
of the grant of the patent:
**23.03.2022 Bulletin 2022/12**

(21) Application number: **12008269.8**

(22) Date of filing: **11.12.2012**

(51) International Patent Classification (IPC):
***A61B 5/00*** *(2006.01)*

(52) Cooperative Patent Classification (CPC):
**A61B 5/0095;** A61B 8/4494; A61B 2562/046;
A61B 2562/14; A61B 2562/146

(54) **Handheld device and method for volumetric real-time optoacoustic imaging of an object**

Tragbare Vorrichtung und Verfahren für volumetrische optoakustische Bildgebung eines Objekts

Dispositif portatif et procédé pour imagerie opto-acoustique en temps réel volumétrique d'un objet

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**18.06.2014 Bulletin 2014/25**

(73) Proprietor: **Helmholtz Zentrum München
Deutsches
Forschungszentrum für Gesundheit und
Umwelt GmbH
85764 Neuherberg (DE)**

(72) Inventors:
• **Razansky, Daniel
8046 Zürich (CH)**

• **Deán-Ben, Xosé Luís
8057 Zürich (CH)**

(74) Representative: **Linsmeier, Josef
Wallinger Ricker Schlotter Tostmann
Patent- und Rechtsanwälte Partnerschaft mbB
Zweibrückenstrasse 5-7
80331 München (DE)**

(56) References cited:
**WO-A1-2011/137385   WO-A1-2012/150721
WO-A2-2011/072198   JP-A- 2004 351 023
US-A- 6 102 857       US-A1- 2002 193 678
US-A1- 2006 184 042   US-A1- 2012 123 256
US-B1- 7 515 948**

EP 2 742 853 B1

**Description**

The present invention relates to a handheld device and method for optoacoustic imaging of an object according to the independent claims.

Optoacoustic imaging is based on the photoacoustic effect, according to which ultrasonic waves are generated due to absorption of electromagnetic radiation by an object, e.g. a biological tissue, and a subsequent thermoelastic expansion of the object.

US 6,102,857 discloses a tomographic imaging system in which optoacoustic signals are acquired by means of a plurality of acoustic sensors positioned along a spiral pattern on a spherical surface which is rotated relative to the sample. In alternative embodiments, rotatable or translatable planar arrangements of evenly spaced acoustic transducers are provided. Because of the required rotation or translation of the transducer arrangements relative to the sample while images are acquired and the need for according drive mechanisms, like motors, the set-up of the disclosed imaging system is complex and therefore not suitable for handheld applications.

US 2010/0249570 A1 discloses a photoacoustic imaging apparatus comprising a box or a wand in which a sparse arrangement of transducer elements, which are spaced apart from one another, is provided. Because of the specific arrangement of transducer elements, it is not possible to efficiently collect three-dimensional optoacoustic images of the object without rotating or translating the apparatus while images are acquired.

US 2002/0193678 A1 discloses a handheld thermoacoustic computed tomography scanner which is placed in physical contact with the skin surface of a patient. A membrane on the outer surface of the scanner is pressed against the skin surface. Resulting thermoacoustic signals are received by a plurality of acoustic sensors arranged about a periphery of the handheld scanner.

WO 2011/072198 A2 discloses a handheld ultrasound sensor. An array of probe beams pass through a coupling medium after reflection and then reflect again to a position sensor. The coupling medium can be made from material with good ultrasound transmission and optical transparency for the desired optical wavelengths of an excitation beam and the probe beams.

US 7,515,948 B1 discloses a method and an apparatus for non-invasively monitoring at least one characteristic of a region of interest in a body which is illuminated by pulsed light to induce a photoacoustic effect. Acoustic radiation is detected, and measured data indicative thereof is generated. The photoacoustic effect is controlled by carrying out at least one of the following: providing the pulsed light of at least two different wavelength, analyzing the measured data to determine time variations of at least one predetermined parameter of a time dependent acoustic signal for each of the wavelengths, and determining oxygen saturation level in the region of interest; and operating a plurality of acoustic elements configured for detection of the acoustic radiation, to thereby define a focal volume for acoustic radiation detection to match dimensions of the region of interest.

JP2004351023 A2 discloses a photoacoustic probe which is composed of a photo-irradiation section using optical fibers and a detector comprising piezoelectric elements, wherein detectors of different diameters are installed at different height positions concentrically on a plurality of concentric regions, wherein the diameters of the concentric regions are larger than the height positions of the concentric regions. The photo-irradiation section is arranged in the center of the concentric regions, and the front surface of the photo-irradiation section is placed at a distance from the object. The invention is based on the problem to provide a handheld device and a corresponding method allowing for improved optoacoustic imaging of an object, in particular allowing for an efficient collection of optoacoustic signals of the object without the need for rotating or translating the device while images are acquired.

This problem is solved by the handheld device and the method according to the independent claims.

The handheld device for optoacoustic imaging of an object according to the invention comprises an irradiation unit for irradiating the object with electromagnetic radiation, in particular light, and a detector unit for detecting acoustic, in particular ultrasonic, waves generated in the object upon irradiation with electromagnetic radiation and is characterized in that the detector unit comprises a two-dimensional array of a plurality of detector elements which are arranged along a first surface.

The method for optoacoustic imaging of an object according to the invention comprises the steps of irradiating the object with electromagnetic radiation, in particular light, and detecting acoustic, in particular ultrasonic, waves generated in the object upon irradiation with electromagnetic radiation and is characterized in that the acoustic waves are detected by a two-dimensional array of a plurality of detector elements which are arranged along a first surface.

The term "handheld device" within the meaning of the present invention relates to any optoacoustic imaging device which is adapted for being seized and held by clasping with fingers and/or a hand in order to position the handheld device onto an object under investigation and/or to move the handheld device by hand relative to the object under investigation, in particular by positioning it onto or moving it along an exterior surface of the object, e.g. the skin of a patient. The term "handheld device" also relates to optoacoustic imaging devices in which only a component thereof, in particular a handheld probe comprising the irradiation unit and/or the detector unit, is adapted for being seized and held by clasping with fingers and/or a hand for same purposes. Preferably, the size of a handheld device or a respective handheld probe within the meaning of the invention is less than 15 cm in width and/or depth and/or height. The term "handheld device" further relates to any optoacoustic imaging device which is designed for acquiring tomographic optoacoustic images at arbitrary orientations of the handheld device or handheld probe, respectively. For example, when acquiring images from the object, the orientation of the handheld device or probe can vary from a vertical up to a vertical down orientation including all orientations in between, in particular a horizontal orientation.

According to an aspect of the invention, a handheld optoacoustic imaging probe is provided, in which a two-dimensional array of ultrasonic detectors is arranged on a, preferably spherical, surface. By this means, the individual detector elements can most efficiently collect signals corresponding to acoustic waves generated in the region of interest, which is preferably located around the center of a sphere. In contrast to systems and methods of the prior art, no movement, in particular no rotation, of the imaging probe and the object relative to each other is necessary in order to ensure an efficient collection of the acoustic waves emerging from the object. Since many imaging scenarios, such as imaging of large areas of human body, do not allow for a full tomographic access to the imaged area from all directions, the handheld device according to the invention is particularly advantageous as the generated acoustic waves are optimally collected from an as broad as possible range of angles, i.e. projections, around the imaged area located inside the object, in particular a living subject.

According to another aspect of the invention, a control unit is provided for controlling the irradiation unit and the detector unit such that the irradiation unit irradiates the object with one or more pulses of electromagnetic radiation, in particular with illumination pulses, and the two-dimensionally arrayed detector elements of the detector unit simultaneously detect acoustic waves emanating from a plurality of locations and/or directions around the object after each pulse of electromagnetic radiation. Preferably, the control unit is designed for forming a three-dimensional image of the object on the basis of the acoustic waves, which are detected by the plurality of detector elements after a single pulse. Thus, by irradiating the object with a series of pulses of electromagnetic radiation an according series of three-dimensional images of the object is formed. Alternatively or additionally, the control unit is designed for forming a three-dimensional image of the object on the basis of acoustic waves which are detected upon a few, in particular up to ten, pulses of electromagnetic radiation. For example, a three-dimensional image is formed on the basis of acoustic waves, which are detected by the detector elements upon each pulse of two, three or four pulses. Due to the specific design and/or arrangement of the detector elements along the first surface according to the invention, a high-resolution three-dimensional image of the object can be obtained upon irradiation of the object by a single pulse or only a few pulses. The term "high-resolution" in this context preferably relates to an effective voxel number per imaged region of the object in the range between 100.000 and 1.000.000. This relatively high number of effective (resolutionlimited) voxels can only be achieved if the space around the imaged object is densely populated with a high number of detection elements so that a full-view (well-posed) inverse (tomographic) problem has been formed. According to this aspect of the invention, high-resolution volumetric optoacoustic images of the object are acquired by means of an irradiation of the object with, e.g., a single laser pulse and simultaneous acquisition of optoacoustic responses, i.e. ultrasonic waves, from as many as possible locations and/or directions (projections) around the object. Since it usually takes on the order of a few tens of microseconds to acquire optoacoustic responses from typical objects or regions of a size of up to a few centimeters, images resulting from a single-pulse acquisition approach will be also significantly less affected by the object's motion, because of e.g. respiration or heartbeats, which in turn further improves spatial resolution and overall imaging accuracy.

Multiple particular advantages exist therefore in achieving a three-dimensional real-time imaging according to the invention. First, dynamic biological processes, such as hemodynamic and neuronal responses or bio-distribution of molecular probes, can be monitored in the entire volume of interest. Second, motion artifacts (due to e.g. breathing or heartbeat of a living object), which could degrade the image quality when imaging living specimen, can be avoided. Finally, real-time performance can obviously reduce the time required for experiments or clinical measurements.

In summary, by means of the handheld device and method according to the invention, a real-time three-dimensional imaging of an object is enabled which allows not only for imaging of dynamic phenomena in the object but also significantly reduces multiple motion-related and limited-view-related image artifacts and thus facilitates quantitative image acquisition. Thus, the invention allows for an improved optoacoustic imaging of an object.

According to a preferred embodiment of the invention, the detector elements cover a major part of the first surface. Preferably, the number and/or dimension of the detector elements are chosen such that they cover at least 70%, preferably at least 85%, of the total area of the first surface. It is also preferred that the first surface is completely covered by the detector elements. Alternatively or additionally, the detector elements are arranged adjacently to each other, i.e. the detector elements adjoin each other and preferably cover the first surface for the most part or the first surface entirely. By this means, acoustic waves generated in the region of interest are detected in a very efficient way so that the detected acoustic response can be maximized, resulting in real-time performance and reduction or elimination of motion-related artifacts and other image inaccuracies resulting from e.g. limited-view tomographic geometries. The latter usually occur in tomographic optoacoustic systems that try to facilitate real-time imaging by e.g. reducing the tomographic problem into two dimensions by cylindrical focusing of the detection elements. This leads to substantial out-of-plane artefacts and quantification errors due to strong absorbers located outside the imaged plane (cross section).

Moreover, anisotropic resolution is rendered due to the focusing of the detection elements while volumetric imaging is performed by scanning and cannot be done in real time.

It is, moreover, preferred that the size of the detector elements is maximized and/or a space between the detector elements is minimized in order to improve signal-to-noise ratio and attain real-time performance without the need for signal averaging.

According to the invention, the detector elements are arranged on concentric rings along the curved first surface. Thus, the efficiency and homogeneity of detection sensitivity among different elements is optimized by achieving an angular symmetry.

The first surface is a curved, in particular a spherically shaped, two-dimensional surface, in particular a concave surface and/or a calotte. By this means, the detector elements arranged on the first surface efficiently collect acoustic waves from the region upon or within the object, which is also located around a center of curvature of the curved two-dimensional surface. For example, if the curved two-dimensional surface is spherically shaped, the detector elements most efficiently collect acoustic waves from a region of the object located around the center of a sphere.

Alternatively, the first surface is a planar surface along which the two-dimensional array of detector elements is arranged. This embodiment is of particular advantage when images of larger volumes shall be acquired, as it ensures a particularly efficient collection of optoacoustic signals emerging from larger objects without the need for translating the device during the image acquisition process. Flat two-dimensional detection arrays are also simpler to manufacture.

In yet another embodiment of the invention, the first surface is a convex surface or comprises a convex surface. By this means, the detection array arranged on the first surface efficiently collects acoustic waves from a wide angle around the array. This embodiment is advantageous for endoscopic imaging inside organs or body cavities, e.g. esophagus, blood vessels or gastrointestinal tract, where very limited tomographic access to the region of interest is possible.

The handheld device comprises a cavity in which a coupling medium, in particular water, is accommodated, wherein the cavity is bounded by the first surface, along which the detector elements are arranged, and by at least one second surface. The second surface is a part of a cover element by means of which the cavity is sealed. In these embodiments, the cavity is formed by the first surface and the second surface, wherein the first surface is preferably formed or constituted by a continuous two-dimensional array of the, e.g. adjacently arranged, detector elements, and wherein the second surface is formed or given by the cover element. By means of these provisions, the detector unit, i.e. the two-dimensional array of the plurality of detector elements, forms a part of the cavity in which the coupling medium is accommodated. Thus, no additional container for the coupling medium is necessary, which facilitates the handling of the handheld device considerably without adversely affecting its optimal real-time performance.

It is preferred that the cover element is a mechanically flexible element, in particular a membrane or a film. The cover element is transparent for at least a part of the electromagnetic radiation. Preferably, at least a part of the cover element has a convex shape, in particular a cushion-like shape. In particular, the cover element is provided on a distal end of the handheld device. Moreover, the cover element is preferably arranged and/or designed such that at least a part of the cover element comes into contact with the object while images are acquired from the object. The transparent membrane is used to enclose the active detection surface while the volume inside the membrane is filled with matching fluid, e.g. water, in order to facilitate optimal acoustic coupling to the imaged object. Due to these provisions, the form of the cavity can - in the region of the cover element - be easily adapted to arbitrary forms of the objects under investigation so that the two-dimensional array of detector elements can be positioned in the respectively required manner relative to the region of interest on or within objects of different shapes. By this means, motion-related and/or out-of-plane artifacts can be reduced or avoided in a reliable and simple manner.

Preferably, the cover element is acoustically and optically matched to the object for an optimal transmission of electromagnetic radiation, in particular light, and the generated acoustic waves. This helps avoiding or at least minimizing losses due to light reflections or reflections of acoustic waves at boundaries between the cover element and the object as well as between the cover element and the coupling medium.

According to another preferred embodiment of the invention, the handheld device is designed such that it can be moved relative to the, preferably non-moving or substantially static, object, while images are acquired from the object. By this means, various regions of interest in the object can be tracked by acquiring their corresponding three-dimensional images in real-time.

In further preferred embodiments of the invention the curvature and/or the size and/or the angular coverage of the first surface is arranged and/or depends on the size of the object and/or the size of a region of interest within the object. Alternatively or additionally, it is preferred that the size of the detector elements and/or the frequency response of the detector elements and/or the shape of the detector elements and/or the detection sensitivity of the detector elements and/or the directivity of the detector elements and/or the orientation of the normal to the surface of the detector elements is chosen such that an effective angular coverage of the detector elements around a region of interest is maximized. Preferably, one or more of these features or parameters are chosen such that the effective angular coverage of the detector elements around a region of interest, their detection bandwidth and signal-to-noise-ratio are maximized. Hereby it is ensured that for any kind of objects, e.g. large areas of the human body or small tissue specimen, acoustic waves are detected by the two-dimensional array of detector elements in the broadest possible solid angle range, i.e. broadest possible range of angles (projections) around the imaged area of the object. Furthermore, by optimizing the shape of the detection surface, the orientation of individual detection elements toward the imaged region can be optimized or the distance of said elements from said region minimized in order to increase the detected optoacoustic responses from all the elements and minimize effects of acoustic refractions and surface mismatches.

From the point of view of pure mathematical tomographic reconstruction problem, it is advantageous to have as many discrete detection elements as possible with the broadest possible coverage around the imaged region in order to effectively resemble a full-view point-detector problem and achieve best possible image quality, spatial resolution and quantification capabilities. On the other hand, increasing the number of elements will correspondingly decrease their size, which will in turn adversely affect the signal-to-noise performance, subsequently increase image noise and, as a result, reduce imaging speed and achievable imaging depth. Thus, the total number of the detection elements is chosen such that both image quality and imaging speed are optimized for the given object, tomographic geometry and imaging requirements of the particular application (imaging speed, quantification, resolution etc).

Moreover, it is preferred that the irradiation unit comprises a light emitting element and/or a light guide, in particular a fiber bundle, which is fed through at least one aperture provided in the first surface. Preferably, the fiber bundle is inserted into an aperture or a hole located in the center of the detection array. This is a simple way to provide an effective irradiation of the imaged region, in particular when the first surface has a concave or spherical shape. For implementations involving flat or convex detection surfaces, for which larger volumes need to be illuminated, it is particularly advantageous that the light emitting element and/or light guide is provided in several apertures in the first surface.

In summary, the preferred embodiments set forth above relate to a detection of acoustic waves with an array of ultrasonic sensors distributed on a spherical surface (or otherwise a surface having shape optimized to the shape and size of the imaged object), which significantly reduces out-of-plane artifacts and helps achieving close to isotropic imaging resolution as well. As the normal to the elements is directed towards the center of the curvature, in particular a sphere, all of the detection elements lying on the curved array efficiently collect optoacoustic signals generated in this region. The optimal orientation of the elements towards the imaged region also maximizes the effective angular coverage of the array, and consequently reduces the effects of limited-view reconstructions and out-of-plane artifacts. Since the magnitude of generated optoacoustic signals is typically very low, especially from deep tissue areas suffering from significant light attenuation, special attention needs to be put regarding detection sensitivity of the individual detection elements. For instance, if detection is performed using piezoelectric elements, their size is increased to guarantee the best possible signal-to-noise ratio.

Of particular importance for quantitative optoacoustic imaging is the effective frequency bandwidth of the detection elements. Broadband detection can be facilitated by using broadband piezoelectric technologies, such as polyvinylidene difluoride (pVDF) films. In order to maximize bandwidth, optical detection of optoacoustic responses is preferably employed, e.g. using Fabry-Perot films, fiber Brag gratings, ring-shaped or spherical resonators. For the optical detection approaches, the detection sensitivity might be further increased by e.g. increasing quality factor of the corresponding resonant structure.

Moreover, illumination is performed by means of a fiber bundle inserted in a cylindrical cavity provided in the two-dimensional array of detector elements. In this way, the optical energy delivered to the imaging region is maximized, contrary to linear arrays with lateral illumination for which only scattered photons provide excitation at locations corresponding to a high-sensitivity of the ultrasonic sensors.

Moreover, the handheld approach including respective preferred embodiments is as well an important aspect of the invention as it allows convenient handling of pre-clinical experiments and clinical measurements. The preferred handheld device uses a transparent membrane in order to allow efficient coupling of optoacoustically generated waves to the ultrasonic detector elements while avoiding direct contact of the imaged object with the coupling medium.

The handheld arrangement can be optimized with respect to additional requirements. For example, in many cases the measurement head is held by hand and not fixed relative to the object, thus the images must be acquired in real time without signal averaging (single pulse acquisition) in order to avoid motion artifacts. Thereby, the arrangement of the illumination set-up and the ultrasonic detectors is crucial. Specifically, maximal detection sensitivity and appropriate orientation of the ultrasonic transducers (maximal tomographic coverage) are essential to efficiently collect the optoacoustic signals generated in the imaging region and ensure real-time performance without the need for signal averaging in order to improve signal-to-noise performance.

Yet, in many cases, no full tomographic access to the object is possible, therefore image reconstruction is challenged by the limited-view problem (only a limited number of positions around the object or the imaged region are accessible). The present three-dimensional acquisition and reconstruction approach helps to considerably mitigate image artifacts associated with those problems as well, helping to make the images more quantitative and identify the correct properties of structures within tissues with respect to e.g. their shape, size, optical absorption and spectral characteristics.

The irradiation unit may use various types of electromagnetic radiation in order to induce an optoacoustic or thermoacoustic effect in the imaged object. This includes short-pulsed lasers, pulsed sources in the microwave and radiofrequency (RF) bands, intensity-modulated light or RF sources.

In another particularly preferred embodiment, a control unit for controlling the irradiation unit and the detector unit is provided such that the irradiation unit illuminates the object with pulsed illumination light and the illumination of the object and the detection of acoustic waves are repeated at least twice with different wavelengths of the illumination light. Additionally or alternatively, the detector elements are controlled such that acoustic waves are detected simultaneously by all of the detector elements. Preferably, the handheld device is arranged such that a three-dimensional image of the object is formed after each illumination pulse, i.e. in real time, without moving the device with respect to the object. This technique, which is called multispectral optoacoustic tomography (MSOT), is very advantageous in optoacoustic imaging of biological tissues as it provides a unique combination of high spatial resolution and rich contrast based on spectrally dependent absorption of light. MSOT enables to simultaneously render images of anatomical, functional and molecular contrast by exciting tissues at several optical wavelengths. Therefore, if the illumination wavelength is further swept in a fast and reliable manner, these embodiments will allow for an acquisition of real-time multispectral optoacoustic tomographic images of the object by means of which a highly sensitive visualization of functional and molecular biomarkers in living tissues with unprecedented anatomical, functional and molecular imaging capabilities is achieved. This will effectively attain a 5D imaging system capable of simultaneously delivering volumetric (3D), time-resolved (4D) and spectrally-enriched (5D) data.

Further advantages, features and examples of the present invention will be apparent from the following description of following figures:

Fig. 1 shows a perspective view of an embodiment of a handheld device according to the invention;

Fig. 2 shows a cross-sectional view of a further embodiment of a handheld device according to the invention;

Fig. 3 shows a cross-sectional view of the embodiment of fig. 2 for illustrating the angle of coverage;

Fig. 4 shows a planar projection of a further embodiment of a handheld device according to the invention comprising a curved two-dimensional array of a plurality of detector elements; and

Fig. 5 shows a spectrum of molar extinction coefficient of oxygenated ($HbO_2$) and deoxygenated (Hb) haemoglobin in the near-infrared.

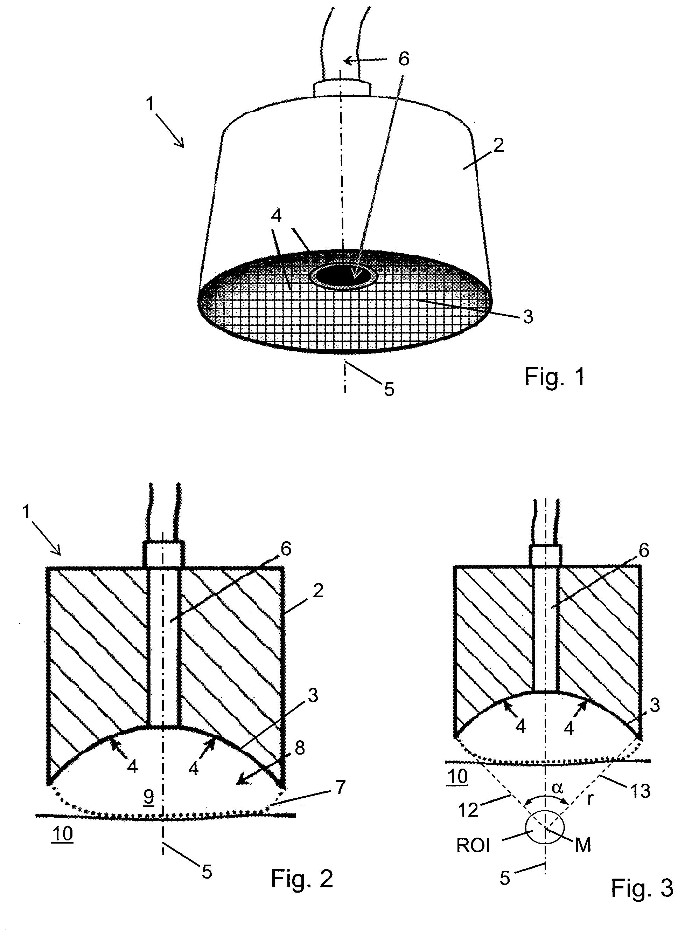

Fig. 1 shows a perspective view of an embodiment of a handheld device 1 according to the invention. The handheld device 1 comprises an essentially cylindrical or cone-shaped body 2 having a front side at which a first surface 3 is provided. The first surface 3 is a curved surface with a concave shape. Preferably, the first surface 3 is a spherically shaped surface which corresponds to the surface of a calotte.

On the first surface 3 a plurality of detector elements 4, in particular ultrasonic detectors, is provided which forms a two-dimensional array of the detector elements 4 for detecting acoustic, in particular ultrasonic, waves emanating from an object under investigation (not shown) and generating according detector signals.

In the example given in fig. 1, the detector elements 4 are schematically represented by a grid-like arrangement of small squares. It is understood, however, that the individual detector elements can have various other shapes and/or sizes and/or orientations. For example, the detector elements can have a rectangular, subrectangular, polygonal or round shape. Preferably, piezoelectric elements are used for detection, wherein the strength of the detected signals can be optimized, i.e. increased, by maximizing the size of the individual detector elements 4 and minimizing the inter-element spacing between the detector elements 4.

Preferably, the total number and/or shape and/or size of the detector elements 4 is chosen such that they cover the first surface 3 entirely or at least a major part, in particular at least 70%, thereof. It is further preferred that the detector elements 4 adjoin each other. By this means, the first surface 3 or at least a major part thereof is formed by or constituted of the two-dimensional array of the detector elements 4.

The elements 4 are arranged symmetrically around a center line 5 of the first surface 3 in order to maintain angular symmetry of the detector elements 4 with respect to the region of interest on or within the object. According to the invention, the elements 4 are arranged on concentric rings around the center line 5.

At the center of the two-dimensional array of detector elements 4, in particular around the center line 5 of the first surface 3, an aperture is provided in which a light guide 6, e.g. a fiber bundle, is arranged for guiding light generated by a light source, in particular a laser light source (not shown), to the handheld device 1 and for illuminating the object under investigation with light. Preferably, the entire volume of interest within the imaged object is illuminated by laser pulses.

According to the invention, the first surface 3 is a curved surface. Alternatively, the first surface 3 can be an essentially planar surface, wherein the above-mentioned elucidations regarding arrangements of detector elements 4 on curved surfaces apply to arrangements of the detector elements 4 on planar surfaces accordingly.

Fig. 2 shows a cross-sectional view of a further embodiment of a handheld device 1 according to the invention. Regarding body 2, first surface 3, detector elements 4 and light guide 6 the elucidations given with respect to fig. 1 apply accordingly.

At a distal end of the handheld device 1 corresponding to the front side of the body 2 a cover element 7 is provided by means of which the front side of the body 2 including the first surface 3 with the detector elements 4 is sealed such that a closed cavity 8 is formed. The cavity 8 is filled with a coupling medium 9 for acoustically coupling acoustic waves emanating from an object 10 to the detector elements 4 at the surface 3 and/or for optically coupling the light emerging from the light guide 6 to the object 10. The coupling medium is chosen such that its acoustic impedance and/or optical refractive index are similar to those of the object 10. In order to avoid reflections and loss of signal, the coupling medium 9 may consist of water or any other optically transparent medium with an acoustic impedance close to soft biological tissues.

The cover element 7 is a mechanically flexible element, e.g. a membrane, a film or a foil of a material which is transparent for light emanating from the light guide 8. The cover element 7 encloses the acoustic coupling medium 9 and facilitates transmission of the optoacoustic waves while preventing a direct contact of the imaged object 10 with the coupling medium 9. In the embodiment shown, the filled cover element 7 has a convex shape so that it can be easily placed onto an object 10 under investigation, irrespective of the spatial orientation of the handheld device 1. For example, reliable image acquisition is guaranteed both in vertical down orientation (shown in fig. 2) of the handheld device 1 or vertical up orientation as well as in any orientations in between, in particular slanted or horizontal orientations. Due to its mechanical flexibility and coupling medium filling, the membrane exhibits a cushion-like shape and behavior by means of which it easily adapts to various surface topologies of objects under investigation.

During image acquisition, the handheld device 1 can be moved relative to the object 10 under investigation, in particular to enable imaging of different regions of interest on or within the object 10. Moreover, the cover element 7, in particular the membrane, can be further coupled to the surface of the object 10 by means of an ultrasonic gel in order to further improve acoustic coupling and to facilitate a movement of the handheld device 1 on the surface of the object 10.

The curvature and/or the size and/or the angular coverage of the first surface 3 is adapted to the size of the object 10 under investigation and/or the size of a region of interest on or within the object 10.

In the sense of the present invention, the term "angular coverage" relates to an angular range within which acoustic waves emanating from a region of the object 10 are detected by the array of detector elements 4 of the handheld device 1.

This is illustrated by the example shown in fig. 3. Dashed lines 12 and 13, which are orthogonal to the detector elements 4 located at a circumferential edge of the first surface 3, intersect at an angle $\alpha$ in an intersection point M, which is located in a region of interest ROI within the object 10. The angular coverage of the array of detector elements 4 corresponds to the angle $\alpha$ which is approximately 90° in the present example. Because the first surface 3 is a spherical surface in this embodiment, the intersection point M corresponds to the center of a sphere having a radius r.

The angular coverage and the radius r of the curvature of the first surface 3, on which the array of detector elements 4 is provided, may vary depending on the particular imaging application. The main guiding principle is to acquire tomographic data from as many angles (i.e. projections) around the imaged volume as possible with the detection elements 4 located as close as possible to the imaged volume for better SNR (signal-to-noise ratio). For example, in the case of a small object, e.g. a small animal or a tissue sample, curvature and/or angular coverage of the first surface are preferably larger than in the case of a large object, e.g. a human body, where the curvature of the first surface is low or can be even planar.

For instance, if the imaged object 10 comprises a relatively small volume (e.g. 1 cm$^3$), which can be fully penetrated by the excitation laser light, a spherical detection array fully enclosing the object from 360° is preferred. Clearly, in a clinical imaging scenario larger volumes are of interest while the excitation light can only effectively penetrate for several centimeters from one side, thus only limited-view acquisition is possible. In this case, imaging depth would play a crucial role in selecting the appropriate angular coverage and radius of curvature of the array. While for superficial targets it would be beneficial and possible to enclose almost 180° around the imaged area with the detector elements 4 arranged along a hemi-spherical array, for deep tissue targets the effective field of view (i.e. angular coverage) of the array will have to be reduced to 90° or less.

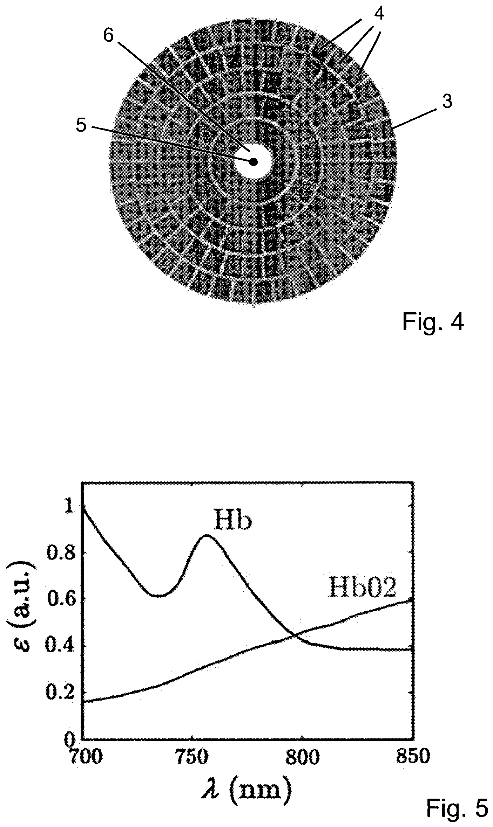

Fig. 4 shows a planar projection of a bottom view on a further embodiment of a handheld device comprising a concave two-dimensional array of a plurality of detector elements 4. In the given example, the array consists of 256 detector elements 4 with an angular coverage of 90°. The detector elements 4 are arranged on five concentric rings around the center line 5 of the array. Preferably, the individual detector elements 4 are arranged and/or shaped such that they cover the first surface 3 entirely. As obvious from this representation, the detector elements 4 are adjacent to each other, wherein each of the detector elements 4 borders on three or more neighboring detector elements 4. In a circularly shaped aperture around the center of the array of detector elements 4 the light guide 6 is provided.

An additional consideration must be given to the size of the detector elements 4. Ultrasound detector elements based on technologies like piezoelectric transducers (PZT) become directional as the size of the element increases. If such an element is placed in close proximity to the imaged object 10 (see fig. 2), it might only detect signals from a small region within the imaged volume, which will in turn reduce quality of the acquired tomographic data. Preferably, the geometry of the array is designed such that each detector element 4 will detect acoustic waves from the entire volume of interest while taking into account also SNR considerations, which require minimal possible distance of the detector elements 4 to the object.

As already set forth above, raw optoacoustic data, i.e. acoustic waves, are simultaneously collected from all the detectors and processed in order to attain images in real time. For image reconstruction several existing algorithms, e.g. back-projection algorithm, can be used.

In the following, a novel three-dimensional model-based approach will be described, which is particularly suitable for accurate extraction of absorption maps from data acquired with the present handheld device or method. Spectral fitting of the images retrieved at several wavelengths is used to provide concentration maps of intrinsic tissue chromophores (such as oxygenated and deoxygenated haemoglobin) as well as extrinsically administered contrast agents.

Image reconstruction from the detector signals corresponding to the detected acoustic waves preferably consists of recovering the distribution of optical absorbed energy, absorption coefficient as well as concentrations of specific chromophores within the imaged region.

In general, the raw optoacoustic signals are related to the optical absorption distribution $H(\mathbf{r}, \lambda)$ for the given laser wavelength $\lambda$. Thus, the generated acoustic pressure $p(r,t)$ for a given position r and instant $t$ is given as a function of $H(\mathbf{r}, \lambda)$ by the Poisson-type integral as

$$p(\mathbf{r},t) = \frac{\Gamma}{4\pi c} \frac{\partial}{\partial t} \int_{S'(t)} \frac{H(\mathbf{r}',\lambda)}{|\mathbf{r} - \mathbf{r}'|} dS'(t). \tag{1}$$

Equation 1 is derived analytically and establishes the optoacoustic forward problem. Image reconstruction consists in solving the inverse problem, i.e., to estimate $H(\mathbf{r}, \lambda)$ as a function of pressure waveforms $p(r,t)$ acquired from multiple measurement locations. A common back-projection approximated analytical formula can be applied in order to calculate $H(\mathbf{r}, \lambda)$. In its discrete form it is given by

$$H(\mathbf{r}'_j, \lambda) = \sum_i \left[ p(\mathbf{r}_i, t_{ij}) - 2t_{ij} \frac{\partial p(\mathbf{r}_i, t_{ij})}{\partial t} \right], \tag{2}$$

where $\mathbf{r}_i$ is the position of $i$-th measuring point, $\mathbf{r}'_j$ is the position of the $j$-th point of the region of interest (ROI) and $t_{ij}=|\mathbf{r}_i-\mathbf{r}'_j|/c$. Equation 2 establishes a very fast reconstruction strategy, which is suitable for displaying the resulting images in real-time during the hand-held scanning procedure.

However, approximations in deriving back-projection formulae may lead to quantitative errors in the reconstructed images, which is particularly important for determining accurate maps of chromophore distribution and other metrics such as blood oxygenation levels.

A different reconstruction approach, also termed the model-based inversion, consists in a numerical inversion of Equation 1. For this, Equation 1 is first discretized by considering a ROI consisting of a grid of points. In this way, the pressure at a point $r_i$ and at an instant $t_j$ can be expressed as a linear combination of the optical absorption at the points of the grid $\mathbf{r}'_k$, i.e.,

$$p(\mathbf{r}_i, t_j) = \sum_{k=1}^{N} a_k^{ij} H(\mathbf{r}'_k, \lambda), \tag{3}$$

where N is the number of points of the grid. By considering P transducer positions and I instants, Equation 3 can be expressed in a matrix form as

$$\mathbf{p} = \mathbf{AH}, \tag{4}$$

being p and H the pressure and the optical absorption in a vector form. The absorbed energy can be subsequently retrieved from Equation 4 by using variety of algorithms, for instance by inverting the model matrix A and multiplying it with the vector of the detected pressure variations p. In case of very large model matrices, such as those obtained for volumetric time-resolved data, the computational demands might be eased on by utilizing sparsity of the model matrix and applying more sophisticated algorithms, e.g. regularization-assisted singular value decomposition (SVD), Moore-Penrose pseudo-inverse or minimization of the least square difference between the theoretical pressure and the measured pressure $\mathbf{p}_m$, i.e.,

$$\mathbf{H}_{sol} = \arg\min_{\mathbf{H}} \| \mathbf{p}_m - \mathbf{A}\mathbf{H} \|^2 + \lambda_T^2 \| \mathbf{H} \|^2 . \tag{5}$$

The term $\lambda_T$ rcorresponds to the regularization parameter, which is needed to obtain a representative (reasonable) solution. Equation 5 can be then solved e.g. by means of an LSQR algorithm to attain distribution of the absorbed light energy.

An alternative approach for handling large-scale inversions is by using multi-resolution methods, such as wavelet, wavelet packets or curvelet-based approaches, capable of separating the problem into a set of multiple smaller problems, each representing a different spatial and time resolution within the object/image.

There are several advantages in using the exact model-based reconstruction approach versus approximated analytical formulas, such as back-projection. First, as mentioned, it avoids inaccuracies involved with analytical approximation formulas, thus improves quantification performance. Second, it allows seamless inclusion of multiple important experimental parameters into the numerical model, such as the particular detection geometry, shape of the individual detection elements and their frequency response, the light pattern upon and within the imaged object, and acoustic heterogeneities in the object. This, in turn, helps to improve quantification and avoid image blurring due to incorrect modeling assumptions. Third, it allows efficient implementation of the said multi-resolution methods for achieving a tremendous acceleration of the reconstruction procedures and the corresponding imaging speed.

For functional and molecular imaging applications, of particular interest is also determining functional tissue parameters (e.g. blood oxygenation levels) and finding distribution of exogenously administered contrast agents based on optoacoustic measurements taken at multiple wavelengths.

For instance, the endogenous optical absorption in biological tissues is mainly due to blood, i.e. the oxygenated ($HbO_2$) and deoxygenated (Hb) haemoglobin, whose molar extinction coefficients in the near infrared are depicted in fig. 5.

Distribution of the absorbed light energy (in arbitrary units) for a set of wavelengths $\lambda_i$ with $i=1,..,n$, is then given by

$$H(\mathbf{r}, \lambda_i) = \phi(\mathbf{r}, \lambda_i)\varepsilon_{Hb}(\lambda_i)C_{Hb}(\mathbf{r}) + \phi(\mathbf{r}, \lambda_i)\varepsilon_{HbO2}(\lambda_i)C_{HbO2}(\mathbf{r}) \tag{6}$$

where $\varepsilon_x(\lambda_i)$ and $C_x(\mathbf{r})$ stand for the molar extinction coefficient and the concentration of a certain chromophore, respectively. Under simplest assumptions, by neglecting the variations of the light fluence $\varphi(\mathbf{r}, \lambda_i)$ with the wavelength, Equation 6 can be expressed in a matrix form as

$$\begin{pmatrix} H(\mathbf{r}, \lambda_1) \\ \vdots \\ H(\mathbf{r}, \lambda_n) \end{pmatrix} = \phi(\mathbf{r}) \begin{pmatrix} \varepsilon_{Hb}(\lambda_1) & \varepsilon_{HbO2}(\lambda_1) \\ \vdots & \vdots \\ \varepsilon_{Hb}(\lambda_n) & \varepsilon_{HbO2}(\lambda_n) \end{pmatrix} \begin{pmatrix} C_{Hb}(\mathbf{r}) \\ C_{HbO2}(\mathbf{r}) \end{pmatrix}, \tag{7}$$

The concentration of oxygenated and deoxygenated haemoglobin can be determined by least-square spectral fitting of the images taken at the $n$ measured wavelengths. This is done with the Moore-Penrose pseudoinverse as

$$\phi(\mathbf{r})\begin{pmatrix} C_{Hb}(\mathbf{r}) \\ C_{HbO2}(\mathbf{r}) \end{pmatrix} = \begin{pmatrix} \varepsilon_{Hb}(\lambda_1) & \varepsilon_{HbO2}(\lambda_1) \\ \vdots & \vdots \\ \varepsilon_{Hb}(\lambda_n) & \varepsilon_{HbO2}(\lambda_n) \end{pmatrix}^{+} \begin{pmatrix} H(\mathbf{r}, \lambda_1) \\ \vdots \\ H(\mathbf{r}, \lambda_n) \end{pmatrix}, \tag{8}$$

Then, the distribution of the oxygenation level $SO_2(\mathbf{r})$ is no longer affected by the illumination light fluence variations and is explicitly given by

$$SO_2(\mathbf{r}) = \frac{C_{HbO2}(\mathbf{r})}{C_{Hb}(\mathbf{r}) + C_{HbO2}(\mathbf{r})} = \frac{\phi(\mathbf{r})C_{HbO2}(\mathbf{r})}{\phi(\mathbf{r})C_{Hb}(\mathbf{r}) + \phi(\mathbf{r})C_{HbO2}(\mathbf{r})} \qquad (9)$$

Due to high level of heterogeneity of realistic biological tissues, the above simple assumptions might however lead to image inaccuracies and lack of quantification. For instance, spatial variations in light fluence due to attenuation are preferably compensated by using e.g. numerical modeling of light propagation in tissues, analytical correction functions or blind correction methods that do not use any models of light propagation but instead sparsely decompose the fluence from the optical absorption coefficient. Another complexity might arise from the lack of precise knowledge about the spectrum of different chrmophores in tissue or otherwise spectrally-dependent light attenuation within the object. In this case, methods based on the ratios between images acquired at different wavelengths, are applied in order to reduce the effect of spectrally-dependent attenuation. In addition, blind spectral methods, such as principal component analysis (PCA) and independent component analysis (ICA), are used in order to retrieve both the spatial distribution maps of the different chromophores as well as their spectral dependence curves.

**Claims**

1. Handheld device (1) for optoacoustic imaging of an object (10) comprising

   - an irradiation unit (6) for irradiating the object (10) with electromagnetic radiation, in particular light,
   - a detector unit (3, 4) for detecting acoustic, in particular ultrasonic, waves which are generated in the object (10) upon irradiation with electromagnetic radiation, and
   - a cavity in which a coupling medium (9) is accommodated, wherein
   - the cavity has a curved first surface (3) and
   - the detector unit (3, 4) comprises a two-dimensional array of a plurality of individual detector elements (4) which are arranged on concentric rings along the curved first surface (3) which is sealed by a cover element (7) such that a closed cavity is formed.

2. Handheld device (1) according to claim 1, wherein the first surface (3) is a concave surface

3. Handheld device (1) according to one of the foregoing claims, wherein the number and/or dimension of the detector elements (4) are chosen such that the detector elements (4) cover at feast 70% of the total area of the first surface (3).

4. Handheld device (1) according to claim 3, wherein the first surface (3) is completely covered by the detector elements (3).

5. Handheld device (1) according to one of the foregoing claims, wherein the detector elements (4) are arranged adjacently to each other.

6. Handheld device (1) according to one of the foregoing claims, wherein the cavity is bounded by the first surface (3), along which the detector elements (4) are arranged, and by at least one second surface, wherein the second surface is a part of the cover element (7) by means of which the cavity is sealed.

7. Handheld device (1) according to one of the foregoing claims comprising a control unit for controlling the irradiation unit (6) and the detector unit (3, 4) such that

   - the irradiation unit (6) irradiates the object (10) with one or more pulses of electromagnetic radiation and
   - the two-dimensionalfy arrayed detector elements (4) of the detector unit (6) simultaneously detect acoustic waves emanating from a plurality of locations and/or directions around the object (10) after each pulse of electromagnetic radiation.

8. Handheld device (1) according to one of the foregoing claims, wherein the cover element (7) is a mechanically flexible membrane or film.

9. Handheld device (1) according to one of the foregoing claims, wherein at least a part of the cover element (7) has a convex shape, in particular a cushion-like shape.

10. Handheld device (1) according to one of the foregoing claims, wherein the irradiation unit comprises a light emitting element and/or a light guide (6), in particular a fiber bundle, which is fed through at least one aperture provided in the first surface (3).

11. Handheld device (1) according to one of the foregoing claims comprising a control unit for controlling the irradiation unit (6) and the detector unit (3, 4) such that the irradiation unit (6) illuminates the object (10) with pulsed illumination light and the illumination of the object (10) and the detection of acoustic waves are repeated at least twice with different wavelengths of the illumination light.

12. Handheld device according to claim 11 arranged such that a three-dimensional image of the object (10) is formed after each illumination pulse, in particular in real time.

13. Method for optoacoustic imaging of an object (10) by

- irradiating the object (10) with electromagnetic radiation, in particular light, and
- detecting acoustic, in particular ultrasonic, waves which are generated in the object (10) upon irradiation with electromagnetic radiation, wherein
the acoustic waves are detected by a two-dimensional array of a plurality of individual detector elements (4) which are arranged on concentric rings along a curved first surface (3) of a cavity, in which a coupling medium (9) is accommodated and which is sealed by a cover element (7) such that a closed cavity is formed.

**Patentansprüche**

1. Handgerät (1) zur optoakustischen Abbildung eines Objekts (10) mit

- einer Bestrahlungseinheit (6) zur Bestrahlung des Objekts (10) mit elektromagnetischer Strahlung, insbesondere Licht,
- einer Detektoreinheit (3, 4) zur Detektion von akustischen Wellen, insbesondere Ultraschallwellen, die bei Bestrahlung mit elektromagnetischer Strahlung im Objekt (10) erzeugt werden, und
- einem Hohlraum, in dem ein Kopplungsmedium (9) untergebracht ist, wobei
- der Hohlraum eine gekrümmte erste Fläche (3) aufweist und
- die Detektoreinheit (3, 4) ein zweidimensionales Array aus einer Vielzahl von einzelnen Detektorelementen (4) aufweist, die auf konzentrischen Ringen entlang der gekrümmten ersten Fläche (3) angeordnet sind, die durch ein Abdeckelement (7) verschlossen ist, so dass ein geschlossener Hohlraum gebildet wird.

2. Handgerät (1) nach Anspruch 1, wobei die erste Fläche (3) eine konkave Fläche ist.

3. Handgerät (1) nach einem der vorhergehenden Ansprüche, wobei die Anzahl und/oder Abmessung der Detektorelemente (4) so gewählt ist, dass die Detektorelemente (4) mindestens 70% der Gesamtfläche der ersten Fläche (3) abdecken.

4. Handgerät (1) nach Anspruch 3, wobei die erste Fläche (3) vollständig von den Detektorelementen (3) bedeckt ist.

5. Handgerät (1) nach einem der vorangehenden Ansprüche, wobei die Detektorelemente (4) aneinander angrenzend angeordnet sind.

6. Handgerät (1) nach einem der vorhergehenden Ansprüche, wobei der Hohlraum durch die erste Fläche (3), entlang derer die Detektorelemente (4) angeordnet sind, und durch mindestens eine zweite Fläche begrenzt ist, wobei die zweite Fläche ein Teil des Abdeckelements (7) ist, mit dem der Hohlraum verschlossen wird.

7. Handgerät (1) nach einem der vorhergehenden Ansprüche mit einer Steuereinheit zur Steuerung der Bestrahlungseinheit (6) und der Detektoreinheit (3, 4) derart, dass

- die Bestrahlungseinheit (6) das Objekt (10) mit einem oder mehreren Impulsen elektromagnetischer Strahlung bestrahlt und
- die zweidimensional angeordneten Detektorelemente (4) der Detektoreinheit (3, 4) nach jedem Impuls elektromagnetischer Strahlung gleichzeitig akustische Wellen detektieren, die von einer Vielzahl von Orten und/oder Richtungen um das Objekt (10) herum ausgehen.

8. Handgerät (1) nach einem der vorangehenden Ansprüche, wobei das Abdeckelement (7) eine mechanisch flexible Membran oder Folie ist.

9. Handgerät (1) nach einem der vorhergehenden Ansprüche, wobei zumindest ein Teil des Abdeckelements (7) eine konvexe Form, insbesondere eine kissenartige Form, aufweist.

10. Handgerät (1) nach einem der vorhergehenden Ansprüche, wobei die Bestrahlungseinheit ein lichtemittierendes Element und/oder einen Lichtleiter (6), insbesondere ein Faserbündel, aufweist, das durch mindestens eine in der ersten Fläche (3) vorgesehene Öffnung geführt ist.

11. Handgerät (1) nach einem der vorhergehenden Ansprüche, umfassend eine Steuereinheit zur Steuerung der Bestrahlungseinheit (6) und der Detektoreinheit (3, 4) derart, dass die Bestrahlungseinheit (6) das Objekt (10) mit gepulstem Beleuchtungslicht beleuchtet und die Beleuchtung des Objekts (10) und die Detektion von akustischen Wellen mindestens zweimal mit unterschiedlichen Wellenlängen des Beleuchtungslichts wiederholt werden.

12. Handgerät nach Anspruch 11, das so eingerichtet ist, dass nach jedem Beleuchtungsimpuls ein dreidimensionales Bild des Objekts (10), insbesondere in Echtzeit, erzeugt wird.

13. Verfahren zur optoakustischen Abbildung eines Objekts (10) durch

- Bestrahlung des Objekts (10) mit elektromagnetischer Strahlung, insbesondere Licht, und
- Detektion von akustischen Wellen, insbesondere Ultraschallwellen, die bei Bestrahlung mit elektromagnetischer Strahlung im Objekt (10) erzeugt werden, wobei
die akustischen Wellen von einem zweidimensionalen Array aus mehreren einzelnen Detektorelementen (4) detektiert werden, die auf konzentrischen Ringen entlang einer gekrümmten ersten Fläche (3) eines Hohlraums angeordnet sind, in welchem ein Kopplungsmedium (9) untergebracht ist und welcher durch ein Abdeckelement (7) derart verschlossen ist, dass ein geschlossener Hohlraum gebildet wird.

## Revendications

1. Dispositif portatif (1) pour imagerie opto-acoustique d'un objet (10) comprenant

- une unité d'irradiation (6) pour irradier l'objet (10) avec un rayonnement électromagnétique, en particulier de la lumière,
- une unité de détection (3, 4) pour détecter des ondes acoustiques, en particulier ultrasoniques, qui sont générées dans l'objet (10) lors d'une irradiation avec un rayonnement électromagnétique, et
- une cavité dans laquelle un milieu de couplage (9) est contenu,
dans lequel
- la cavité a une première surface (3) incurvée et
- l'unité de détection (3, 4) comprend un réseau bidimensionnel d'une pluralité d'éléments de détection (4) individuels qui sont agencés sur des anneaux concentriques le long de la première surface (3) incurvée qui est fermée hermétiquement par un élément de couverture (7) de sorte qu'une cavité close soit formée.

2. Dispositif portatif (1) selon la revendication 1, dans lequel la première surface (3) est une surface concave.

3. Dispositif portatif (1) selon l'une des revendications précitées, dans lequel le nombre et/ou la dimension des éléments de détection (4) sont choisis de sorte que les éléments de détection (4) couvrent au moins 70 % de la superficie totale de la première surface (3).

4. Dispositif portatif (1) selon la revendication 3, dans lequel la première surface (3) est complètement couverte par les éléments de détection (3).

**5.** Dispositif portatif (1) selon l'une des revendications précitées, dans lequel les éléments de détection (4) sont agencés de manière adjacente les uns par rapport aux autres.

**6.** Dispositif portatif (1) selon l'une des revendications précitées, dans lequel la cavité est bordée par la première surface (3), le long de laquelle les éléments de détection (4) sont agencés, et par au moins une seconde surface, dans lequel la seconde surface est une partie de l'élément de couverture (7) au moyen duquel la cavité est fermée hermétiquement.

**7.** Dispositif portatif (1) selon l'une des revendications précitées comprenant une unité de commande pour commander l'unité d'irradiation (6) et l'unité de détection (3, 4) de sorte que

- l'unité d'irradiation (6) irradie l'objet (10) avec une ou plusieurs impulsions de rayonnement électromagnétique et
- les éléments de détection (4) disposés en réseau de manière bidimensionnelle de l'unité de détection (3, 4) détectent simultanément des ondes acoustiques émanant d'une pluralité d'emplacements et/ou de directions autour de l'objet (10) après chaque impulsion de rayonnement électromagnétique.

**8.** Dispositif portatif (1) selon l'une des revendications précitées, dans lequel l'élément de couverture (7) est une membrane ou un film flexible mécaniquement.

**9.** Dispositif portatif (1) selon l'une des revendications précitées, dans lequel au moins une partie de l'élément de couverture (7) a une forme convexe, en particulier une forme semblable à un coussin.

**10.** Dispositif portatif (1) selon l'une des revendications précitées, dans lequel l'unité d'irradiation comprend un élément électroluminescent et/ou un conduit de lumière (6), en particulier un faisceau de fibres, qui est alimenté à travers au moins une ouverture fournie dans la première surface (3).

**11.** Dispositif portatif (1) selon l'une des revendications précitées comprenant une unité de commande pour commander l'unité d'irradiation (6) et l'unité de détection (3, 4) de sorte que l'unité d'irradiation (6) éclaire l'objet (10) avec une lumière d'éclairage pulsée et l'éclairage de l'objet (10) et la détection d'ondes acoustiques sont répétés au moins deux fois avec des longueurs d'ondes différentes de la lumière d'éclairage.

**12.** Dispositif portatif selon la revendication 11 agencé de sorte qu'une image tridimensionnelle de l'objet (10) soit formée après chaque impulsion d'éclairage, en particulier en temps réel.

**13.** Procédé pour imagerie opto-acoustique d'un objet (10) en

- irradiant l'objet (10) avec un rayonnement électromagnétique, en particulier de la lumière, et
- détectant des ondes acoustiques, en particulier ultrasoniques, qui sont générées dans l'objet (10) lors d'une irradiation avec un rayonnement électromagnétique,

dans lequel
les ondes acoustiques sont détectées par un réseau bidimensionnel d'une pluralité d'éléments de détection (4) individuels qui sont agencés sur des anneaux concentriques le long d'une première surface (3) incurvée d'une cavité, dans laquelle un milieu de couplage (9) est contenu et qui est fermée hermétiquement par un élément de couverture (7) de sorte qu'une cavité close soit formée.

Fig. 1

Fig. 2

Fig. 3

Fig. 4

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6102857 A
- US 20100249570 A1
- US 20020193678 A1
- WO 2011072198 A2
- US 7515948 B1
- JP 2004351023 A